# EUROPEAN PATENT SPECIFICATION

(11) **EP 1 817 013 B1**
(45) Date of publication and mention of the grant of the patent: **04.06.2008**
(21) Application number: 05824194.4
(22) Date of filing: 17.11.2005
(51) Int. Cl.: A61K 9/52

(54) **ENTERIC COATED BEAD COMPRISING IXABEPILONE**
MAGENSAFTRESISTENT BESCHICHTETES PELLET MIT IXABEPILON
BILLE A ENROBAGE ENTERIQUE COMPRENANT DE L'IXABEPILONE

(30) Priority: 18.11.2004 US 628970 P
(43) Date of publication of application: 15.08.2007
(73) Proprietor: Bristol-Myers Squibb Company, Princeton, NJ 08543-4000 (US)
(72) Inventor: ULLAH, Ismat, Cranbury, New Jersey 08512 (US); WILEY, Gary, James, Maine, New York 13802 (US)
(74) Representative: Beacham, Annabel Rose
(86) International application number: PCT/US2005/041736
(87) International publication number: WO 2006/055740

(56) References cited:
- WO-A-20/04012714
- US-A1- 2002 177 615
- US-A1- 2003 017 210
- US-A1- 2003 220 378
- US-B1- 6 537 579

## Description

### FIELD OF THE INVENTION

The present invention generally relates to an enteric coated bead comprising Ixabepilone. A method is provided for preparing the enteric coated bead. Also, a method of treating cancer or other proliferative diseases using the enteric coated bead is provided.

### BACKGROUND OF THE INVENTION

Ixabepilone is a macrocyclic compound having the structure: 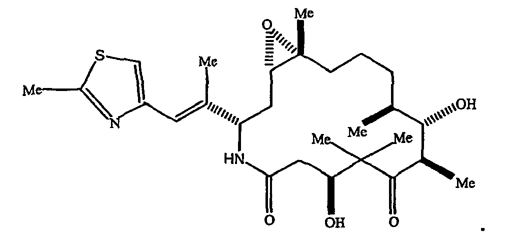 Ixabepilone exerts microtubule-stabilizing effects similar to TAXOL^{®} and therefore exhibits cytotoxic activity against rapidly proliferating cells, such as occur in cancer and other hyperproliferative cellular diseases (*See* Angew. Chem. Int. Ed. Engl., Vol. 35, No. 13/14, 1996 and D.M. Bollag, Exp. Opin. Invest. Drugs, 6(7): 867-873, 1997).

Before Ixabepilone can be used to treat diseases in patients, however, it must be formulated into pharmaceutical compositions that can be administered to the patient; for example, into a dosage form suitable for oral, mucosal (*e*.*g*., nasal, sublingual, vaginal, buccal, or rectal), parenteral (*e.g.*, subcutaneous, intravenous, bolus injection, intramuscular, or intraarterial), or transdermal administration. Formulations for oral administration are particularly preferred since they are more convenient and easier to administer than other formulations. Also, the oral route of administration avoids the pain and discomfort of parenteral administration. Oral administration is preferred by patients and results in better patient compliance with dosing schedules.

Oral administration involves passage of Ixabepilone through the stomach, where it is exposed to low pH gastric fluids, and then passage into the small intestine, where Ixabepilone is absorbed into the bloodstream. Transit time through the stomach is approximately two hours. The pH of the stomach is approximately 1 to 3. The small intestine, which includes the duodenum, jejunum, and the ileum, has pH values for these regions of approximately 5 to approximately 7.2. However, Ixabepilone is acid labile with maximum stability in aqueous solution in the pH range between 7 and 8.5. Thus, Ixabepilone is susceptible to degradation, decomposition, or deactivation in aqueous solution, particularly in acidic solutions, such as those found in the stomach. In oral administration, the bioavailability of Ixabepilone is dependent upon minimizing loss of Ixabepilone in the acid conditions encountered during passage through the stomach.

US 2003/0220378 describes the oral and parenteral administration of epothilones. The compounds are administered in liquid form with an acid neutralizing buffer.

U.S. Patent No. 6,576,651 discloses a method for oral administration of Ixabepilone. The method comprises orally administering Ixabepilone, and orally administering one or more pharmaceutically acceptable acid neutralizing buffers. The acid neutralizing buffer may be administered prior to, concurrently, or after the administration of Ixabepilone. The disclosed method allows the delivery of Ixabepilone to a mammal while reducing or avoiding the degradation, decomposition, or deactivation of Ixabepilone by the gastrointestinal system, particularly by gastric fluid in the stomach. However, raising the pH of the stomach can cause stomach upset and indigestion. Further, oncology patients often need to take other medicines, for some of which alkaline stomach conditions may not be desirable. Desired are an oral dosage form and a method for the oral administration of Ixabepilone that does not require neutralization of the stomach acid.

One method to protect Ixabepilone from contact with gastric contents is to encase the Ixabepilone particles with an enteric coating. Ixabepilone is typically prepared as a fine powder and prior to the application of an enteric coating, the fine particles of Ixabepilone need to be granulated to prepare larger drug containing particles. However, Ixabepilone is a potent drug, and any dry process involving Ixabepilone, including a dry granulation process, would require special handling during the manufacture of the dosage form. For example, a containment facility and manufacturing equipment with special engineering controls to reduce or remediate dust formed during dry processing operations may be required. Such facility and equipment would entail significant planning and a large capital investment. Desired is a dosage form that may be prepared by a method that minimizes handling of dry Ixabepilone.

A wet process for preparing an enteric coated bead of Ixabepilone would reduce or eliminate dusting of dry Ixabepilone. However, Ixabepilone is susceptible to degradation, decomposition, or deactivation in the presence of water and/or heat. Desired is a wet process, in particular an aqueous process, for preparing an enteric coated bead comprising Ixabepilone.

In accordance with the present invention, a method is provided for preparing an enteric coated bead, wherein the method reduces or eliminates dusting of Ixabepilone powder. Further, an enteric coated bead is provided, that is suitable for oral administration of Ixabepilone without requiring coadministration of an acid neutralizing buffer.

### SUMMARY OF THE INVENTION

The present invention relates to an enteric coated bead comprising: a coated particle comprising a base particle and an active ingredient layer disposed on the base particle, wherein the active ingredient layer comprises Ixabepilone, or a pharmaceutically acceptable salt, solvate, clathrate, hydrate, or prodrug thereof, and one or more binders; and an enteric coating encapsulating the coated particle.

Also provided are a process for preparing the enteric coated bead, a capsule comprising the enteric coated beads, and a method of treating cancer or other proliferative diseases comprising orally administering the enteric coated bead to a patient in need thereof.

### DETAILED DESCRIPTION OF THE INVENTION

### Definitions

The following are definitions of various terms used herein to describe the present invention.

The term "Ixabepilone" encompasses Ixabepilone or a pharmaceutically acceptable salt, solvate, clathrate, hydrate, or prodrug thereof throughout this description.

As used herein, "substantially free of moisture" refers to a composition comprising less than 4 weight % water, preferably less than 3 weight % water, and more preferably, less than 2 weight % water, based on the weight of the composition. Examples of suitable ranges for "substantially free of moisture" include from zero to less than 4 weight %, preferably from zero to about less than 3 weight %, and more preferably from zero to less than 2 weight %, based on the weight of the composition.

Preparation, formulation, and use of Ixabepilone is described in U.S. Patent 6,365,749 B1; U.S. Patent 6,518,421 B1; U.S. Patent 6,576,651 B1; U.S. Patent No. 6,605,599 B1; U.S. 6,686,380 B1; U.S. Patent Application Publication 20030073677 A1; U.S. Patent Application Publication 20040024032 A1; and U.S. Patent Application Publication 2004026254 A1.

The present invention relates to an enteric coated bead comprising Ixabepilone, which is suitable for oral administration to a patient. The enteric coated bead comprises a coated particle, which comprises an active ingredient layer disposed on a base particle; and an enteric coating encapsulating the coated particle. The active ingredient layer comprises Ixabepilone and at least one binder. The enteric coating is capable of protecting the Ixabepilone, which is susceptible to degradation, decomposition, or deactivation during exposure to acidic conditions, from low pH gastric fluids typically encountered during passage through the stomach into the intestine. The enteric coating is capable of minimizing or preventing exposure of the active ingredient layer to stomach acid. This prevents Ixabepilone from being released in the stomach or the stomach acid from penetrating through to the active ingredient layer. Upon passage of the enteric coated bead to the small intestine, the enteric coating partially or completely dissolves in the higher pH conditions encountered in the intestine, leading to the release of Ixabepilone, and its passage to the bloodstream of the patient.

The enteric coated bead comprises a coated particle encapsulated by an enteric coating. The coated particle comprises a base particle, which provides a seed particle for the application of the active ingredient layer. The base particle comprises a pharmaceutically acceptable material that is capable of carrying the active ingredient layer. Generally, the base particle comprises, for example, a pharmaceutically inert material, such as, for example, sugar, starch, microcrystalline cellulose, lactose, or combinations thereof Optionally, the base particle may further comprise one or more active agents. The shape of the base particle is typically spherical or semispherical, although other shapes are contemplated. Average diameters for the base particles are typically, for example, in the range of from about 0.1 millimeters to about 5 millimeters. Examples of suitable base particles include Nu-Pareil^{™} Sugar Spheres NF (Chr. Hansen, Inc., WI) and Celphere^{™} microcrystalline cellulose spheres (Asahi Kasei Kogyo Kabushiki Kaisha Corp., Japan). Typically, the enteric coated bead comprises, for example, from 10 to 80 weight % base particle, preferably from 15 to 70 weight % base particle, and more preferably from 20 to 65 weight % base particle, based on the weight of the enteric coated bead. Preferably, the base particle is substantially free of moisture. More preferably, the base particle comprises less than 3 weight % water, based on the weight of base particle.

The coated particle comprises an active ingredient layer disposed on the base particle. The active ingredient layer is applied to the base particle and may form a surface layer on the surface of the base particle, absorb into the base particle, or a combination thereof. The active ingredient layer may be completely or partially distributed on, in, and/or beneath the surface of the base particle. Preferred is an active ingredient layer that is uniformly disposed on the surface of the base particle.

The active ingredient layer comprises Ixabepilone, or a pharmaceutically acceptable salt, solvate, clathrate, hydrate, or prodrug thereof. In addition to Ixabepilone, the active ingredient layer may optionally comprise at least one additional active agent, such as an anticancer drug. In one embodiment, the active ingredient layer may comprise a mixture of Ixabepilone and a pharmaceutically acceptable salt, solvate, clathrate, hydrate, or prodrug of Ixabepilone. For example, the active ingredient layer may comprise a mixture of Ixabepilone and a clathrate of Ixabepilone. Suitable levels of Ixabepilone include, for example, those in the range of from 0.1 weight % to 10 weight %, preferably from 0.2 weight % to 5 weight %, and more preferably from 0.5 weight % to 4 weight %, based on the weight of the enteric coated bead.

The active ingredient layer also comprises binder. The binder may be employed to improve adhesion of Ixabepilone to the base particle and/or to provide cohesion of the active ingredient layer. Materials suitable as binders include, for example, starch; gelatin; sugars such as sucrose, glucose, dextrose, molasses, and lactose; natural and synthetic gums such as acacia, sodium alginate, methyl cellulose, carboxymethylcellulose, and polyvinylpyrrolidone (PVP) polymers and copolymers such as polyvinylpyrrolidone/polyvinyl acetate (PVP-PVA) copolymers; celluloses such as ethyl cellulose, hydroxypropyl cellulose, or hydroxypropyl methylcellulose; polyethylene glycol; and waxes. For example, suitable commercially available materials include Avicel^{™} PH 101, Avicel^{™} RC 591, and Avicel^{™} CL611 cellulose crystallite materials, (FMC Corp., PA). One or more different binders may be used in the active ingredient layer. One or more optional ingredients that may be included in the active ingredient layer are, for example; buffers, antifoam agents, and plasticizers. The enteric coated bead may comprise, for example, from 2 to 80 weight % of the active ingredient layer, preferably from 10 to 70 weight % of the active ingredient layer, and more preferably from 20 to 60 weight % of the active ingredient layer, based on the weight of the enteric coated bead. Preferably, the active ingredient layer is substantially free of moisture.

The enteric coated bead has an enteric coating that encapsulates the coated particle. The enteric coating is insoluble or has low solubility in acid solutions characteristic of gastric fluids encountered in the stomach, such pH values of less than about 3. At higher pH values, such as those encountered in the small intestine, the enteric coating dissolves to allow the release of Ixabepilone. Examples of the higher pH values encountered in the small intestine include pH values of greater than 4.5, preferably pH values of greater than 5, and most preferably pH values in the range of from 5 to 7.2.

Suitable materials for forming the enteric coating, include, for example, enteric coating polymers, such as, for example, hydroxypropyl methylcellulose phthalate, polyvinyl acetate phthalate, cellulose acetate phthalate, acrylic acid copolymers, hydroxypropyl methylcellulose acetate succinate, and methacrylic acid copolymers. One example of a suitable methacrylic acid copolymer is Eudragit^{™} L-30-D 55 aqueous copolymer dispersion, which comprises an anionic copolymer derived from methacrylic acid and ethyl acrylate with a ratio of free carboxyl groups to the ethyl ester groups of approximately 1:1, and a mean molecular weight of approximately 250,000, and is supplied as an aqueous dispersion containing 30 weight % solids. Eudragit^{™} L-30-D 55 aqueous copolymer dispersion is supplied by Röhm-Pharma Co., Germany.

The enteric coated bead may comprise, for example, from 5 to 55 weight % of the enteric coating, preferably from 10 to 45 weight % of the enteric coating, and more preferably from 15 to 40 weight % of the enteric coating, based on the weight of the enteric coated bead. Preferably, the enteric coating is substantially free of moisture.

The enteric coating optionally comprises other materials, such as plasticizers, colorants, antifoam agents, and anti-adherents.

The enteric coated bead optionally comprises one or more subcoat layers that are situated between the base particle and the active ingredient layer, or the active ingredient layer and the enteric coating. A subcoat layer may be employed to minimize contact between Ixabepilone contained in the active ingredient layer and an enteric coating comprising acid groups, such as methacrylic acid copolymer. For example, the enteric coated bead may comprise from 0.1 to 10 weight % of the subcoat layer, preferably from 0.5 to 5 weight % of the subcoat layer, and more preferably from 2 to 4 weight % of a subcoat layer, based on the weight of the enteric coated bead. Suitable materials to form the subcoat layer include starch; gelatin; sugars such as sucrose, glucose, dextrose, molasses, and lactose; natural and synthetic gums such as acacia, sodium alginate, methyl cellulose, carboxymethylcellulose, and polyvinylpyrrolidone (PVP) polymers and copolymers such as PVP-PVA copolymers; celluloses such as ethylcellulose, hydroxypropyl cellulose, and hydroxypropyl methyl cellulose; polyethylene glycol, and waxes. The subcoat layer may further comprise one or more plasticizers, such as polyethylene glycol, propylene glycol, triethyl citrate, triacitin, diethyl phthalate, tributyl sebecate, or combinations thereof.

In one embodiment, the enteric coated bead comprises a subcoat layer interposed between the active ingredient layer and the enteric coating. In this embodiment, the enteric coated bead may comprise from 0.1 to 10 weight % of the subcoat layer, preferably from 0.5 to 5 weight % of the subcoat layer, and more preferably from 2 to 4 weight % of a subcoat layer, based on the weight of the enteric coated bead. Preferably, the subcoat layer is substantially free of moisture.

The enteric coated bead optionally comprises other materials such as flavoring agents, preservatives, or coloring agents as may be necessary or desired.

In one non-limiting embodiment, the enteric coated bead is substantially free of moisture. Preferably, the enteric coated bead comprises less than 4 weight % water, preferably less than 3 weight % water, and more preferably, less than 2 weight % water, based on the weight of the enteric coated bead.

The enteric coated bead may be contacted with a hydrophobic material such as talc, magnesium stearate, or fumed silica to form a hydrophobic layer on the surface of the enteric coated bead. The hydrophobic layer is useful to reduce agglomeration of the individual enteric coated beads and/or to reduce static during the handling of the enteric coated beads.

In one embodiment of the invention, the enteric coated bead comprises: a coated particle and an enteric coating encapsulating the coated particle.

In a second embodiment of the invention, the enteric coated bead comprises: a coated particle comprising a base particle, a subcoat disposed on the base particle, and the active ingredient layer disposed on the subcoat; and an enteric coating encapsulating the coated particle.

In a third embodiment of the invention, the enteric coated bead comprises: a coated particle; a subcoat disposed on the coated particle; and an enteric coating encapsulating the coated particle.

In a fourth embodiment of the invention, the enteric coated bead comprises: a coated particle comprising a base particle, a first subcoat disposed on the base particle, and the active ingredient layer disposed on the subcoat; a second subcoat disposed on the coated particle; and an enteric coating encapsulating the coated particle.

In a fifth embodiment of the invention, the enteric coated bead comprises: a coated particle in which the base particle also comprises a second pharmaceutically active ingredient; and an enteric coating encapsulating the coated particle. The enteric coated bead of this embodiment optionally comprises a first subcoat situated between the base particle and the active ingredient layer; and/or a second subcoat situated between the coated particle and the enteric coating. The second pharmaceutically active ingredient may be Ixabepilone, or a pharmaceutically acceptable salt, solvate, clathrate, hydrate, or prodrug thereof. Alternatively the second pharmaceutically active ingredient may be another active agent, such as a second anticancer agent.

The enteric coated beads of this invention may be prepared by a process that reduces the exposure of Ixabepilone to moisture, heat, or a combination of moisture and heat. Such a process ensures high potency and good uniformity of the active pharmaceutical agent, since Ixabepilone is susceptible to degradation or decomposition in the presence of water, and especially a combination of moisture and heat.

In one aspect of the present invention, a process is provided for preparing the enteric coated bead, comprising:
a) providing base particles;
b) applying an active ingredient mixture and binder to the base particles, wherein the active ingredient mixture comprises:
   i) Ixabepilone, or a pharmaceutically acceptable salt, solvate, clathrate, hydrate, or prodrug thereof, and
   ii) solvent, water, or a mixture thereof;
c) drying the base particles having application of the active ingredient mixture to provide coated particles; and
d) applying enteric coating to the coated particles to provide the enteric coated beads.

In the present process to prepare the enteric coated bead of this invention, the active ingredient mixture may also comprise the binder, thus allowing co-application of a single mixture. Alternatively, the active ingredient mixture and a solution comprising the binder may be premixed immediately prior to application.

The active ingredient mixture comprises Ixabepilone in solvent, water, or a mixture thereof. The active ingredient mixture may be a solution comprising Ixabepilone dissolved in the solvent, water, or mixture thereof. Alternatively, the active ingredient mixture may be an active agent suspension comprising particles of Ixabepilone dispersed in the solvent, water, or mixture thereof. Suitable solvents include, for example, alcohols such as methanol, ethanol, n-propanol, and isopropanol; and acetone. The active ingredient mixture may be prepared by admixing Ixabepilone in solvent, water, or a mixture thereof. Optionally, the binder may be included in the active ingredient mixture. Ixabepilone and the optional binder may be combined in any order with the solvent, water, or mixture thereof Typically, mixing is required to minimize any localized concentrations of Ixabepilone or the optional binder in the solvent, water, or mixture thereof. Mixing may be provided by a mechanical device, such as a magnetic or overhead stirrer.

In one embodiment, the enteric coated bead of this invention is prepared by applying an active ingredient suspension and binder to the base particles. Preferably, the active ingredient suspension is an aqueous active ingredient suspension comprising the particles of Ixabepilone dispersed in an aqueous medium. The aqueous medium comprises greater than 50 weight % water and optionally, one or more water miscible solvents, based on the weight of the aqueous medium. Preferably the aqueous medium comprises at least 65 weight % water, more preferably at least 75 weight % water, and most preferably at least 85 weight % water, based on the weight of the aqueous medium. The aqueous suspension of the Ixabepilone particles provides a reduction in contact between the aqueous medium and Ixabepilone, compared to a solution of Ixabepilone, and thus decreases the rate of degradation or decomposition of Ixabepilone. The aqueous active ingredient suspension may be prepared by admixing Ixabepilone particles and optionally, the binder, in water and optionally, water miscible solvent. The Ixabepilone particles and the optional binder may be combined with the water and/or the optional water miscible solvent in any order. Typically, mixing is required to disperse the Ixabepilone particles and minimize any localized concentrations of the Ixabepilone particles or the optional binder. Suitable size ranges for the Ixabepilone particles include, for example, from less than 1000 microns, preferably less than 500 microns, and more preferably less than 250 microns. The Ixabepilone particles may be amorphous or crystalline. Preferably, the Ixabepilone particles are crystalline. Examples of crystalline forms of Ixabepilone, such as Form A and Form B, are disclosed in U.S. Patent 6,689,802. The active ingredient suspension may comprise from 1 to 50 weight % Ixabepilone particles, preferably from 2 to 30 weight % Ixabepilone particles, and more preferably from 3 to 20 weight % Ixabepilone particles, based on the weight of the active ingredient suspension. Preferably, the active ingredient suspension has a pH in the range of from 6 to 9, more preferably in the range of from about 6.5 to about 8, and most preferably in the range of from 6.5 to 7.5. The active ingredient suspension may optionally comprise other ingredients, such as buffers; dispersing agents such as surfactants or low molecular weight polymers; antifoaming agents, and pH adjusting agents such as acids and bases.

The binder may be provided as a solution or dispersion in water.

In one embodiment, the active ingredient mixture may comprise, for example, from 1 to 30 weight % of the at least one binder, preferably from 2 to 20 weight % of the at least one binder, and more preferably from 3 to 10 weight % of the at least one binder, based on the weight of the active ingredient mixture.

The active ingredient mixture and the binder solution may be applied to the base particles as a spray or a stream while base particles are in motion. The conditions are preferably controlled to minimize particle agglomeration of the base particles. Subsequently, the solvent and/or water is removed from the applied active ingredient mixture leaving the coated particles having the active ingredient layer disposed on the base particle.

The enteric coating may be applied to the coated particles by applying a mixture of the enteric coating as a spray or stream while the coated particles are in motion. The enteric coating mixture may be a solution or a suspension. The conditions are preferably controlled to minimize particle agglomeration. The enteric coating mixture comprises the enteric coating material in an aqueous or nonaqueous solvent or mixture thereof. Suitable solvents include, for example, alcohols such as methanol and isopropanol; and acetone. Mixtures of solvents or mixtures of water and one or more water miscible solvents may be used. The enteric coating material may be dissolved into the solvent to provide a solution, or alternatively, may be a dispersion of particles, to provide a suspension, such as an aqueous copolymer dispersion. Typically, the enteric coating mixture may comprise, for example, from 5 to 50 weight % of the enteric coating material, and preferably from 10 to 40 weight % of the enteric coating material, based on the weight of the enteric coating mixture.

Drying to remove the solvent and/or water may be applied during and/or after application of the enteric coating mixture. In one embodiment, the drying conditions include an inlet drying air temperature in the range of from 20°C to 70°C, an inlet air humidity of less than 50% relative humidity, a product bed temperature in the range of from 20°C to 40°C, and air flow that is sufficient to remove the free water vapor.

A fluid bed spraying apparatus, a tangential spray coater, or a rotating pan type coater may be employed to spray the active agent suspension onto the base particles, and/or to spray the enteric coating mixture onto the coated particle.

A fluid bed coater is an apparatus that can fluidize particles such as beads while simultaneously spraying on and drying a film coat. The fluidizing air is heated to the desired temperature and the air flow adjusted to the flow rate for proper fluidization and drying. A pan coater is an apparatus in which particles are tumbled in a pan while spraying a film coat. Simultaneously air of the proper temperature and airflow passes through the bed of particles to dry the applied film coat.

In one aspect of the invention, a capsule comprising a multitude of the enteric coated beads is provided, suitable for oral administration of Ixabepilone. The capsule is prepared by filling a capsule shell, such as a gelatin capsule shell, with the enteric coated beads. The capsule allows for easier swallowing during oral administration of the enteric coated beads. Optionally, the capsule comprises at least one hydrophobic material to reduce agglomeration of the individual enteric coated beads in the capsule and/or to reduce static during the loading of the enteric coated beads into the capsule. Generally, the amount of the optional hydrophobic material is preferably kept to a level where it is just enough to prevent particle sticking after the capsule shell has dissolved, but not too much to retard dissolution. Examples of suitable hydrophobic materials include talc, magnesium stearate, stearic acid, glyceryl behenate, hydrogenated cottonseed oil, trimyristin, triplamitan, tristearin, and fumed silica. Examples of commercially available hydrophobic materials include Lubrital^{™} additive (Penwest Pharmaceutical Co., NJ); Dynasan^{™} 114, Dynasan^{™} 116, and Dynasan^{™} 118 additives (Sasol North America, TX); and Compritol^{™} 888 ATO additive (Gattefosse Co., France). A preferred hydrophobic material is talc.

### UTILITY

Ixabepilone is useful as a microtubule-stabilizing agent. Ixabepilone is useful in the treatment of a variety of cancers and other proliferative diseases including, but not limited to, the following:
- carcinoma, including that of the bladder, breast, colon, kidney, liver, lung, ovary, pancreas, stomach, cervix, thyroid, and skin, including squamous cell carcinoma;
- hematopoietic tumors of lymphoid lineage, including leukemia, acute lymphocytic leukemia, acute lymphoblastic leukemia, B-cell lymphoma, T-cell lymphoma, Hodgkins lymphoma, non-Hodgkins lymphoma, hairy cell lymphoma, and Burketts lymphoma;
- hematopoietic tumors of myeloid lineage, including acute and chronic myelogenous leukemias and promyelocytic leukemia;
- other tumors, including melanoma, seminoma, teratocarcinoma, neuroblastoma, and glioma;
- tumors of the central and peripheral nervous system, including astrocytoma, neuroblastoma, glioma, and schwannomas;
- tumors of mesenchymal origin, including fibrosarcoma, rhabdomyoscaroma, and osteosarcoma; and
- other tumors, including melanoma, xeroderma pigmentosum, keratoacanthoma, seminoma, thyroid follicular cancer, and teratocarcinoma.

Ixabepilone is useful for treating patients who have been previously treated for cancer, as well as those who have not previously been treated for cancer. The methods and compositions of this invention, including the enteric coated beads, can be used in first-line and second-line cancer treatments. Furthermore, the enteric coated beads are useful for treating refractory or resistant cancers.

Ixabepilone will inhibit angiogenesis, thereby affecting the growth of tumors and providing treatment of tumors and tumor-related disorders. Such anti-angiogenesis properties will also be useful in the treatment of other conditions responsive to anti-angiogenesis agents including, but not limited to, certain forms of blindness related to retinal vascularization, arthritis, especially inflammatory arthritis, multiple sclerosis, restinosis, and psoriasis.

Ixabepilone will induce or inhibit apoptosis, a physiological cell death process critical for normal development and homeostasis. Alterations of apoptotic pathways contribute to the pathogenesis of a variety of human diseases. The subject compounds, as modulators of apoptosis, will be useful in the treatment of a variety of human diseases with aberrations in apoptosis including, but not limited to, cancer and precancerous lesions, immune response related diseases, viral infections, kidney disease, and degenerative diseases of the musculoskeletal system.

The enteric coated beads may also be formulated or co-administered with other therapeutic agents that are selected for their particular usefulness in administering therapies associated with the aforementioned conditions. The enteric coated beads may be formulated with agents to prevent nausea, hypersensitivity, and gastric irritation, such as anti-emetics, and H₁ and H₂ antihistamines. The above therapeutic agents, when employed in combination with Ixabepilone, may be used in those amounts indicated in the Physicians' Desk Reference (PDR) or as otherwise determined by one of ordinary skill in the art.

Furthermore, the enteric coated beads may be administered in combination with other anti-cancer and cytotoxic agents and treatments useful in the treatment of cancer or other proliferative diseases. Administration of the enteric coated beads may be prior to, during, or after the administration of the other anti-cancer agents, cytotoxic agents and/or the treatments useful in the treatment of cancer or other proliferative diseases. Especially useful are anti-cancer and cytotoxic drug combinations wherein the second drug chosen acts in a different manner or different phase of the cell cycle, *e*.*g*., S phase, than Ixabepilone which exert its effects at the G₂-M phase. Examples of classes of anti-cancer and cytotoxic agents include, but are not limited to, alkylating agents, such as nitrogen mustards, alkyl sulfonates, nitrosoureas, ethylenimines, and triazenes; antimetabolites, such as folate antagonists, purine analogues, and pyrimidine analogues; antibiotics, such as anthracyclines, bleomycins, mitomycin, dactinomycin, and plicamycin; enzymes, such as L-asparaginase; farnesyl-protein transferase inhibitors; hormonal agents, such as glucocorticoids, estrogens/antiestrogens, androgens/antiandrogens, progestins, and luteinizing hormone-releasing hormone anatagonists, octreotide acetate; microtubule-disruptor agents, such as ecteinascidins or their analogs and derivatives; microtubule-stabilizing agents such as paclitaxel (TAXOL^{®}), docetaxel (TAXOTERE^{®}); plant-derived products, such as vinca alkaloids, epipodophyllotoxins, and taxanes; topoisomerase inhibitors; prenyl-protein transferase inhibitors; and miscellaneous agents such as, hydroxyurea, procarbazine, mitotane, hexamethylmelamine, platinum coordination complexes such as cisplatin and carboplatin; and other agents used as anti-cancer and cytotoxic agents such as biological response modifiers, growth factors, immune modulators, and monoclonal antibodies. The enteric coated beads may also be used in conjunction with radiation therapy.

Representative examples of these classes of anti-cancer and cytotoxic agents include, but are not limited to, mechlorethamine hydrochlordie, cyclophosphamide, chlorambucil, melphalan, ifosfamide, busulfan, carmustin, lomustine, semustine, streptozocin, thiotepa, dacarbazine, methotrexate, thioguanine, mercaptopurine, fludarabine, pentastatin, cladribin, cytarabine, fluorouracil, doxorubicin (including salts such as doxorubicin hydrochloride), daunorubicin, idarubicin, bleomycin sulfate, mitomycin C, actinomycin D, safracins, saframycins, quinocarcins, discodermolides, vincristine, vinblastine, vinorelbine tartrate, etoposide (including salts such as etoposide phosphate), teniposide, paclitaxel, tamoxifen, estramustine, estramustine phosphate sodium, flutamide, buserelin, leuprolide, pteridines, diyneses, levamisole, aflacon, interferon, interleukins, aldesleukin, filgrastim, sargramostim, rituximab, BCG, tretinoin, irinotecan hydrochloride, betamethosone, capecitabine, gemcitabine hydrochloride, altretamine, and topoteca and analogs or derivatives thereof.

Other examples of these classes of anticancer and cytotoxic agents include, but are not limited to, cisplatin, carboplatin, carminomycin, aminopterin, methotrexate, methopterin, ecteinascidin 743, porfiromycin, 5-fluorouracil (5-FU), 6-mercaptopurine, gemcitabine, cytosine arabinoside, paclitaxel, doxorubicin, daunorubicin, mitomycin C, podophyllotoxin or podophyllotoxin derivatives such as etoposide, etoposide phosphate or teniposide, melphalan, vinblastine, vincristine, leurosidine, vindesine, and leurosine. It is to be understood ixabepilone may be administered in combination with particular anticancer and cytotoxic agents falling within these classes of agents, for example, Ixabepilone may be administered in combination with any 5-FU agents, and/or prodrugs thereof, including without limitation capecitabine (XELODA^{®}).

Further examples of anti-cancer and other cytotoxic agents include the following: cyclin dependent kinase inhibitors as found in WO 99/24416; and prenyl-protein transferase inhibitors as found in WO 97/30992 and WO 98/54966.

Without wishing to be bound to any mechanism or morphology, it is expected that the enteric coated beads, which comprise Ixabepilone, could also be used to treat conditions other than cancer or other proliferative diseases. Such conditions include, but are not limited to viral infections such as herpesvirus, poxvirus, Epstein-Barr virus, Sindbis virus, and adenovirus; autoimmune diseases such as systemic lupus erythematosus, immune mediated glomerulonephritis, rheumatoid arthritis, psoriasis, inflammatory bowel diseases, and autoimmune diabetes mellitus; neurodegenerative disorders such as Alzheimer's disease, AIDS-related dementia, Parkinson's disease, amyotrophic lateral sclerosis, retinitis pigmentosa, spinal muscular atrophy, and cerebellar degeneration; AIDS; myelodysplastic syndromes; aplastic anemia; ischemic injury associated myocardial infarctions; stroke and reperfusion injury; restenosis; arrhythmia; atherosclerosis; toxin-induced or alcohol induced liver diseases; hematological diseases such as chronic anemia and aplastic anemia; degenerative diseases of the musculoskeletal system such as osteoporosis and arthritis; aspirin-sensitive rhinosinusitis; cystic fibrosis; multiple sclerosis; kidney diseases; and cancer pain.

The effective amount of Ixabepilone may be determined by one of ordinary skill in the art, and includes exemplary dosage amounts for a human for treatment of cancer or other proliferative diseases of from about 1 to 500 mg/m², which may be administered in a single dose or in the form of individual divided doses, such as from 1 to 4 times per day. For example, metastatic breast cancer may be treated by administering a dose of up to 40 mg/m² of Ixabepilone once per day every 21 days. It will be understood that the specific dose level and frequency of dosage for any particular subject may be varied and will depend upon a variety of factors including the metabolic stability and length of action of Ixabepilone, the species, age, body weight, general health, sex and diet of the subject, the mode and time of administration, rate of excretion, drug combination, and severity of the particular condition.

Preferred subjects for treatment include animals, most preferably mammalian species such as humans, and domestic animals such as dogs, cats and the like, subject to the aforementioned disorders.

Typically Ixabepilone is administered until the patient shows a response, for example, a reduction in tumor size, or until dose limiting toxicity is reached. One of ordinary skill in the art will readily know when a patient shows a response or when dose limiting toxicity is reached. The common dose limiting toxicities associated with Ixabepilone may include, but are not limited to, fatigue, arthralgia/myalgia, anorexia, hypersensitivity, neutropenia, thrombocytopenia, or neurotoxicity.

One of ordinary skill in the art would readily know how to convert doses from mg/kg to mg/m² given either or both the height and or weight of the patient (See, e.g., http://www.fda.gov/cder/cancer/animalframe.htm).

As discussed above, the enteric coated beads are administered orally. The method of this invention encompasses dosing protocols such as once or twice daily. The oral administration may be daily for a continuous period, weekly, or may be for an intermittent period, such as every 3 to 4 weeks between administration.

In one embodiment, a dosage of from 1 to 50 mg/m² is administered daily.

In a different embodiment, a dosage of from 2 to 150 mg/m² is administered weekly, such as daily for two days followed by 5 days with no oral administration of the enteric coated bead.

In a still different embodiment, a dosage of from 10 to 300 mg/m² is administered over a period of 3 to 4 weeks, such as, for example, daily for one day followed by a period of 20 days with no administration of the enteric coated bead.

### EXAMPLES

The following examples are provided, to further illustrate the present invention.

### Example 1

### Preparation of Active Ingredient Suspension

An active ingredient suspension was prepared containing Ixabepilone, [1S-[1R*,3R*(E),7R*,10S*,11R*,12R*,16S*]]-7,11-Dihydroxy-8, 8,10,12,16-pentamethyl-3-[1-methyl-2-(2-methyl-4-thiazolyl)ethenyl]-4-aza-17-oxabicyclo[14.1.0]heptadecane-5,9-dione. First, 2.783 g Tris powder (tris(hydroxymethyl aminomethane)), 500 ml water, and 1 N HCl were mixed to provide a 0.046 M Tris buffer solution having a pH of 8.1. Next, a mixture of 43.5 g Tris buffer solution (43.5 g) and 2.5 g Opadry^{™} Clear Coat powder (Colorcon, Inc., PA), as the binder, was prepared. To this mixture, 4 g of Ixabepilone, as crystals, was added and stirred for approximately 30 minutes to provide the active ingredient suspension. The active ingredient suspension was passed through a 60 mesh screen to remove any agglomerates.

### Preparation of Coated Particles

The coated particles were prepared by applying the active ingredient suspension onto base particles. The base particles were 18/20 mesh sugar beads, (Sugar Spheres, NF particles, (Chr. Hansen, Inc., WI)) having particle diameters of greater than 0.85 mm and less than 1 mm.

The active ingredient suspension was applied to the base particles by spraying using a fluid bed processor that was set up as a Wuster spray coating system. The spray coating system included an Aeromatic-Fielder MP-MICRO™ fluid bed processor (Niro Inc., Maryland) equipped with a 0.8 mm spray tip. The fluid bed processor was charged with 90 g of the sugar beads and then preheated to approximately 50°C for several minutes. The active ingredient suspension was applied to the base particles with the following application and drying parameters: a spray rate of 1.1 g/minute with a spray atomization pressure of 1.8 bar (180 kilopascals), an inlet temperature of 68 °C, an outlet temperature of 32°C, a product bed temperature of 32°C, and a fan speed of 4 m³/hr. During the application process, the active ingredient suspension was slowly stirred.

After application of the active ingredient suspension was completed, the inlet temperature was maintained at the final inlet temperature until the bed product temperature reached 40°C.

The resulting coated particles contained 2.75 weight % of Ixabepilone, based on the weight of the coated particle.

### Application of Subcoat Layer

A subcoat was applied to the coated particles. The subcoat solution was prepared by combining 5 g Opadry^{™} Clear Coat powder and 95 g water and stirring until a clear solution was obtained.

In the subcoating procedure, the fluid bed processor used to prepare the coated particles was employed. The fluid bed processor, which contained 80 g of the coated particles, was preheated to approximately 50°C for several minutes. The subcoat layer was applied using the application and drying parameters disclosed hereinabove to the preparation of the coated particles. During the application process, the subcoated solution was slowly stirred. After application of the subcoat solution was completed, the inlet temperature was maintained at the final inlet temperature until the bed product temperature reached 40 °C. The resulting coated particles, which had a subcoat, contained approximately 2 weight % subcoat, based on the total weight of the resulting coated particles.

### Application of Enteric Coating

An enteric coating was applied onto the coated particles having a subcoat. The enteric coating solution was prepared by first filtering Eudragit^{™} L30D55 polymer dispersion (Röhm GmbH and Co., Darmstadt, Germany) through a 60 mesh screen. Eudragit^{™} L30D55 polymer dispersion is an aqueous suspension containing methacrylic acid copolymer. The filtered Eudragit polymer dispersion (200g) was diluted with 89.5 g water. Next, 9 g diethyl phthalate was added to the diluted Eudragit polymer dispersion, followed by the addition of 9.5 g of 1 N NaOH solution. The pH of the resulting enteric coating solution was 5.0±0.1.

In the enteric film coating procedure, the fluid bed processor used to prepare the coated particles was employed. The fluid bed processor, which contained 70 g of the coated particles, was preheated to approximately 50°C for several minutes. The enteric coating solution was applied using the following application and drying parameters: 0.8 mm spray tip, 1.1 g/minute spray rate, spray atomization pressure was 1.8 bar, inlet temperature 65°C, outlet temperature 30°C, product bed temperature 30°C, and fan speed of 3.5 m³/hr. During the application process, the enteric coating solution was slowly stirred. After application of the enteric coating solution was completed, the inlet temperature was maintained at the final inlet temperature until the bed product temperature reached 40 °C. The resulting enteric coated beads had an average particle diameter of 1 mm.

Table 1 lists the composition of the enteric coated beads prepared in this example. The composition is reported as weight % of each ingredient based on the total weight of the enteric coated bead.

**Table 1**

| **Ingredients** | **% w/w** |
|---|---|
| **A. Coated particles** | |
| Sugar spheres | 55.49 |
| Ixabepilone | 1.60 |
| Binder | 1.00 |
| tris (hydroxymethyl) aminomethane | 0.10 |

| **B. Subcoat Layer** | |
|---|---|
| Subcoat | 1.80 |

| **C. Enteric Coating** | |
|---|---|
| methacrylic acid copolymer | 34.59 |
| Diethyl phthalate | 5.19 |
| NaOH | 0.22 |
| Total | 100.00 |

### Example 2

### Preparation of Active Ingredient Suspension

An active ingredient suspension was prepared containing Ixabepilone, [1S-[1R*,3R*(E),7R*,10S*,11R*,12R*,165*]]-7,11-Dihydroxy-8, 8,10,12,16-pentamethyl-3-[1-methyl-2-(2-methyl-4-thiazolyl)ethenyl]-4-aza-17-oxabicyclo[14.1.0]heptadecane-5,9-dione. First, 2.7832 g Tris powder (tris(hydroxymethyl aminomethane)), 484.5 g water, and 12.7 g 1 N HCl were mixed to provide a 0.046 M Tris buffer solution having a pH of 8.1 ±0.1. Next, a mixture of 33.6 g of Tris buffer solution and 4 g of Ixabepilone, as crystals, was added and stirred. To this mixture 2.4 g Opadry^{™} Clear Coat powder (Colorcon, Inc., PA), as the binder, was added and stirred for approximately 30 minutes to provide the active ingredient suspension. The active ingredient suspension was passed through a 60 mesh screen to remove agglomerates.

### Preparation of Drug Coated Particles

The coated particles were prepared by applying the active ingredient suspension onto base particles. The base particles were 14/18 mesh sugar beads, (Sugar Spheres, NF particles, (Chr. Hansen, Inc., WI)) having particle diameters of greater than 1 mm and less than 1.4 mm.

The active ingredient suspension was applied to the base particles by spraying using a fluid bed processor that was set up as a Wuster spray coating system. The spray coating system included an Aeromatic-Fielder MP-MICRO™ fluid bed processor (Niro Inc., Maryland) equipped with a 0.8 mm spray tip. The fluid bed processor was charged with 70 g of the sugar beads and then preheated to 30-50°C. The active ingredient suspension was applied to the base particles with the following application and drying parameters: a spray rate of 1.0 to 1.2 g/minute with a spray atomization pressure of 1.8 bar (180 kilopascals), an inlet temperature of 65-70 °C, an outlet temperature of 28-32°C, a product bed temperature of 27-32°C, and a fan speed of 3.8 to 4.2 m³/hr. During the application process, the active ingredient suspension was slowly stirred.

After application of the active ingredient suspension was completed, the inlet temperature was maintained at the final inlet temperature until the bed product temperature reached 38-42°C. The other alternative is to immediately continue the spray with the subcoat and dry at the end of that process.

### Application of Subcoat Layer

A subcoat was applied to the drug coated particles. The subcoat solution was prepared by combining 8 g Opadry^{™} Clear Coat powder and 92 g water and stirring until a clear solution was obtained.

In the subcoating procedure, the fluid bed processor used to prepare the coated particles was employed. Drug coated particles (65 g), were preheated to approximately 30-50°C in the fluid bed processor. The subcoat layer was applied using the application and drying parameters disclosed hereinabove to the preparation of the coated particles. During the application process, the subcoated solution was slowly stirred. After application of the subcoat solution was completed, the inlet temperature was maintained at the final inlet temperature until the bed product temperature reached 38-42 °C.

### Application of Enteric Coating

An enteric coating was applied onto the drug coated particles having a subcoat. The enteric coating solution was prepared by first filtering Eudragit^{™} L30D55 polymer dispersion (Röhm GmbH and Co., Darmstadt, Germany) through a 60 mesh screen. Eudragit^{™} L30D55 polymer dispersion is an aqueous suspension containing methacrylic acid copolymer. The filtered Eudragit polymer dispersion (133.34 g) was diluted with 55.61 g water. Next, 6 g diethyl phthalate was added to the diluted Eudragit polymer dispersion, followed by the addition of 5.05 g of 1 N NaOH solution. The pH of the resulting enteric coating solution was 5.0±0.1.

In the enteric film coating procedure, the fluid bed processor used to prepare the drug coated particles was employed. The fluid bed processor, which contained 65 g of the sub-coated particles, was preheated to 30-50°C. The enteric coating solution was applied using the following application and drying parameters: 0.8 mm spray tip, 1.0 to 1.2 g/minute spray rate, spray atomization pressure was 1.8 bar, inlet temperature 65- 70°C, outlet temperature 30-36°C, product bed temperature 28-32°C, and fan speed of 3.9- 4.1 m³/hr. During the application process, the enteric coating solution was slowly stirred. After application of the enteric coating solution was completed, the inlet temperature was maintained at the final inlet temperature until the bed product temperature reached 38-42 °C. The resulting enteric coated beads had an estimated average particle diameter of 1.4 mm.

Table 2 lists the composition of the enteric coated beads prepared in this example. The composition is reported as weight % of each ingredient based on the total weight of the enteric coated bead.

**Table 2**

| **Ingredients** | **% w/w** |
|---|---|
| **A. Coated particles** | |
| Sugar spheres | 71.0538 |
| Ixabepilone | 2.2220 |
| Opadry^{®} Clear | 1.3332 |
| tris (hydroxymethyl) aminomethane (solids) | 0.1039 |
| 1N HCl (solids) | 0.0171 |

| **B. Subcoat Layer** | |
|---|---|
| Opadry Clear | 3.1100 |

| **C. Enteric Coating** | |
|---|---|
| methacrylic acid copolymer (Eudragit^{®} L30D55) | 19.0017 |
| Diethyl phthalate | 2.8501 |
| 1N NaOH (solids) | 0.1082 |

| **D. Talc Addition** | |
|---|---|
| talc | 0.2000 |
| **Total** | 100.00 |

### Example 3

Enteric coated beads comprising Ixabepilone were prepared as described below. A summary of the enteric coated bead compositions is shown in Table 3.

**Table 3**

| Example | Precoat layer | active ingredient layer | subcoat layer | enteric coating |
|---|---|---|---|---|
| 3.1 | yes-buffered | buffered | yes | yes |
| 3.2 | no | buffered | yes | yes |
| 3.3 | no | buffered | no | yes |
| 3.4 | no | nonbuffered | yes | yes |
| 3.5 | no | nonbuffered | no | yes |

All coatings were prepared in Aeromatic-Fielder Type MP Micro fluid bed unit fitted with bottom spray. The coating set-up was as follows: charge (50-90 g), column setting (1cm), spray nozzle diameter (0.8 mm), atomization pressure (1.8 bar), spray rate (0.9-1.1 b/minute), fan speed 3.5-4.0 m³/hr), inlet temperature (58-72°C), bed temperature (30-33°C). At the end of each coating step, product was dried further until a bed temperature of approximately 40°C was reached.

The size of the sugar beads was 18/20 mesh. The coating solutions and suspensions used were as follows:

Buffered Opadry Pre-coat: This consisted of 8% (w/w) solution of Opadry® Clear (YS-1-19025-A) in 0.046 M Tris buffer (pH 8.1 ± 0.1). Applied to obtain ∼4% weight gain.

Opadry Sub coat: This consisted of 8% (w/w) solution of Opadry® Clear in MilliQ water. Applied to obtain -4 % weight gain

Buffered Drug Coat: This consisted of 5% (w/w) solution of Opadry® Clear in 0.046 M Tris buffer (pH 8.1 ± 0.1) containing 12% (w/w) Ixabepilone. Applied to obtain ∼3.7 % weight gain.

Un-buffered Drug Coat: This consisted of 5% (w/w) solution of Opadry® Clear in MilliQ water and containing 12% (w/w) Ixabepilone. Applied to obtain ∼3.7 % weight gain.

Enteric Coat: This consisted of 66.67 % (w/w) Eudragit® L30D-55 (30% solids), 3% diethyl phthalate in MilliQ water and the suspension pH was adjusted to 5.0 ± 0.1 with 1N NaOH. Applied to obtain ∼35 % weight gain

The enteric coated beads of Examples 3.1-3.5 were placed in scintillation glass vials and stored at 40°C for 8 weeks. The enteric coated beads were assayed by HPLC using the following assay procedure:
Column: YMC-Pack Pro C8, 150*4.6 mm, 3mm. S/N
Mobile Phase A: 10mM NH₄OAc in water: acetonitrile (90:10) (NHaOAc, Sigma)
Mobile Phase B: 10mM NH₄OAc in water: acetonitrile (30:70) (ACN: EM Science)
Flow Rate: 1.5 mL/min
Detection: UV at 240 nm
Injection Volume: 10mL
Needle washing sol: Water:acetonitrile (50:50)
Column Temperature: Ambient
Sample Temperature: 4°C

| Gradient: | % Mobile | % Mobile |
|---|---|---|
| Time (min) | Phase A | Phase B |
| 0-2 | 80 | 20 |
| 2-36 | 80-69.5 | 20-30.5 |
| 36-51 | 69.5-20 | 30.5-80 |
| 51-56 | 20-80 | 80-20 |
| 56-71 | 80 | 20 |

Diluent: acetonitrile (EM Science)
Standard Solution: (0.2mg/mL, Ixabepilone, Purity 99.3%)

The standard solution was prepared by weighting ∼50.0 mg Ixabepilone into a 250 mL volumetric flask, followed by the addition of 100 mL diluent. The mixture was sonicated for about 5 minutes or until the solid material was dissolved. The solution was stored at 4°C for up to 7 days.

The enteric coated beads were prepared for assay using a Tablet Process Workstation (Caliper Lifescience, Hopkinton, MA). Sample preparation: (0.2mg/mL).

**Table 4**

| Assays of Examples 3.1-3.5 after Storage at 40°C for 8 weeks | | | | |
|---|---|---|---|---|
| Example | Description | %Ixabepilone Remaining | Main Degradants | Total Degradants and Impurities |
| 3.1 | Buffered Precoat Buffered Drug Coat Subcoat Enteric Coat | 95.2 | 2.16 | 2.54 |
| 3.2 | Buffered Drug Coat Subcoat Enteric Coat | 98.4 | 1.29 | 1.75 |
| 3.3 | Buffered Drug Coat Enteric Coat | 94.5 | 2.44 | 2.93 |
| 3.4 | Unbuffered Drug Coat Subcoat Enteric Coat | 98.5 | 1.27 | 1.74 |
| 3.5 | Unbuffered Drug Coat Enteric Coat | 97.1 | 2.75 | 3.26 |

## Claims

1. An enteric coated bead comprising:
a) coated particle comprising:
i) a base particle; and
ii) an active ingredient layer disposed on said base particle, wherein said active ingredient layer comprises:
1) a compound having the formula 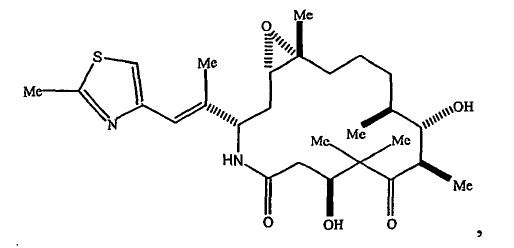 or a pharmaceutically acceptable salt, solvate, clathrate, hydrate, or prodrug thereof; and
2) at least one binder; and
b) an enteric coating encapsulating said coated particle.

2. The enteric coated bead according to claim 1 comprising, based on weight of said enteric coated bead,
a) from 10 to 80 weight % of said base particle;
b) from 0.1 to 50 weight % of said active ingredient layer; and
c) from 5 to 55 weight % of said enteric coating.

3. The enteric coated bead according to claim 1, wherein said enteric coated bead is substantially free of moisture.

4. The enteric coated bead according to claim 1 wherein said at least one binder is starch, gelatin, sucrose, glucose, dextrose, molasses, modified dextrins, lactose, acacia, sodium alginate, potassium alginate, methyl cellulose, hydroxymethyl cellulose, hydroxypropyl methylcellulose, hydroxypropyl cellulose, hydroxyethyl cellulose, guar gum, xanthan gum, polyvinylpyrrolidone, poly(vinylpyrrolidone-vinyl acetate) copolymers, or a mixture thereof

5. The enteric coated bead according to claim 1 wherein said enteric coating comprises hydroxypropyl methylcellulose phthalate, polyvinyl acetate phthalate, cellulose acetate phthalate, hydroxypropyl methylcellulose acetate succinate, or methacrylic acid copolymer.

6. The enteric coated bead according to claim 1 further comprising a subcoat layer interposed between said active ingredient layer and said enteric coating.

7. The enteric coated bead according to claim 6 comprising from 0.1 to 10 weight % of said subcoat layer, based on weight of said enteric coated bead.

8. The enteric coated bead according to claim 6 wherein said subcoat layer is starch, gelatin, sucrose, glucose, dextrose, molasses, modified dextrins, lactose, acacia, sodium alginate, potassium alginate, methyl cellulose, hydroxymethyl cellulose, hydroxypropyl methylcellulose, hydroxypropyl cellulose, hydroxyethyl cellulose, guar gum, xanthan gum, polyvinylpyrrolidone, poly(vinylpyrrolidone-vinyl acetate) copolymers, or a mixture thereof.

9. The enteric coated bead according to claim 1 having a diameter in the range of from 0.5 millimeter to 7 millimeters.

10. A capsule comprising enteric coated beads according to claim 1.

11. The capsule according to claim 10 further comprising at least one hydrophobic material selected from talc, magnesium stearate, stearic acid, glyceryl behenate, hydrogenated cottonseed oil, trimyristin, triplamitan, tristearin, and fumed silica.

12. A process for preparing enteric coated beads, comprising:
a) providing base particles;
b) applying an active ingredient mixture and binder to said base particles, wherein said active ingredient mixture comprises
i) a compound having the structure: 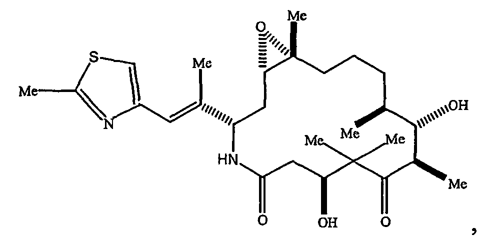 or a pharmaceutically acceptable salt, solvate, clathrate, hydrate, or prodrug thereof, and
ii) solvent, water, or a mixture thereof;
c) drying said base particles having application of said active ingredient mixture to provide coated particles; and
d) applying an enteric coating to said coated particles to provide said enteric coated beads.

13. The process according to claim 12 wherein said active ingredient mixture is an aqueous active ingredient suspension comprising particles of said compound in water.

14. The process according to claim 13 wherein said aqueous active ingredient suspension has a pH in the range of from 6 to 9.

15. The process according to claim 14 wherein said aqueous active ingredient suspension further comprises buffer.

16. The process according to claim 13 wherein said aqueous active ingredient suspension is applied to said base particles in step b) by spraying in a fluid bed spraying apparatus.

17. The process according to claim 16 wherein said base particles having application of said aqueous active ingredient suspension are dried in step c) at a temperature in the range of from 25°C to 35°C in said fluid bed spraying apparatus.

18. The process according to claim 12 wherein said enteric coated bead is substantially free of moisture.

19. The process according to claim 12 wherein said base particles in step a) are substantially free of moisture.

20. An enteric coated bead according to claim 1 for use in treating cancer or other proliferative diseases in a mammal.

21. The enteric coated bead according to claim 20 wherein said mammal is human and said effective amount is in a range of from 1 to 500 mg/m² of said compound, or a pharmaceutically acceptable salt, solvate, clathrate, hydrate or prodrug thereof.

22. The enteric coated bead according to claim 20 wherein said cancer is breast cancer or lung cancer.

23. A capsule comprising a multitude of said enteric beads according to claim 1, for use in treating cancer or other proliferative diseases in a mammal.

## Patentansprüche

1. Magensaftresistent beschichtetes Pellet umfassend:
a) ein beschichtetes Partikel umfassend:
i) ein Basispartikel; und
ii) eine aus aktiven Bestandteilen bestehende Schicht, die auf das Basispartikel aufgebracht ist, wobei die aus aktiven Bestandteilen bestehende Schicht Folgendes umfasst:
1) eine Verbindung, die die Formel 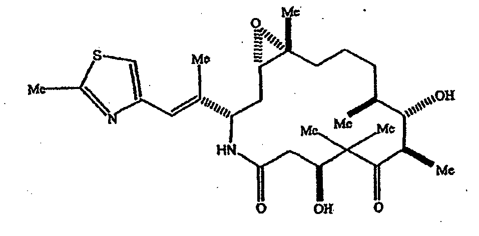 aufweist oder ein pharmazeutisch akzeptables Salz, Solvat, Clathrat, Hydrat oder Prodrug derselben; und
2) mindestens ein Bindemittel; und
b) eine magensaftresistente Beschichtung, die das beschichtete Partikel verkapselt.

2. Magensaftresistent beschichtetes Pellet nach Anspruch 1, umfassend, auf das Gewicht des magensaftresistent beschichteten Pellets bezogen,
a) 10 bis 80 Gew.-% des Basispartikels;
b) 0,1 bis 50 Gew.-% der aus aktiven Bestandteilen bestehenden Schicht; und
c) 5 bis 55 Gew.-% der magensaftresistenten Beschichtung.

3. Magensaftresistent beschichtetes Pellet nach Anspruch 1, wobei das magensaftresistent beschichtete Pellet im Wesentlichen von Feuchtigkeit frei ist.

4. Magensaftresistent beschichtetes Pellet nach Anspruch 1, wobei mindestens ein Bindemittel Stärke, Gelatine, Saccharose, Glukose, Dextrose, Melasse, modifizierte Dextrine, Lactose, Gummi arabicum, Natriumalginat, Kaliumalginat, Methylcellulose, Hydroxymethylcellulose, Hydroxypropylmethylcellulose, Hydroxypropylcellulose, Hydroxyethylcellulose, Guaran, Xanthangummi, Polyvinylpyrrolidon, Poly(vinylpyrrolidon-Vinylacetat)-Copolymere oder eine Mischung derselben ist.

5. Magensaftresistent beschichtetes Pellet nach Anspruch 1, wobei die magensaftresistente Beschichtung Hydroxypropylmethylcellulosephthalat, Polyvinylacetatphthalat, Celluloseacetatphthalat, Hydroxypropylmethylcelluloseacetatsuccinat oder Methacrylsäurepolymer umfasst.

6. Magensaftresistent beschichtetes Pellet nach Anspruch 1, des Weiteren umfassend eine Unterschicht, die zwischen der aus aktiven Bestandteilen bestehenden Schicht und der magensaftresistenten Beschichtung eingelagert ist.

7. Magensaftresistent beschichtetes Pellet nach Anspruch 6, umfassend 0,1 bis 10 Gew.-% der Unterschicht, auf das Gewicht des magensaftresistent beschichteten Pellets bezogen.

8. Magensaftresistent beschichtetes Pellet nach Anspruch 6, wobei die Unterschicht Stärke, Gelatine, Saccharose, Glukose, Dextrose, Melasse, modifizierte Dextrine, Lactose, Gummi arabicum, Natriumalginat, Kaliumalginat, Methylcellulose, Hydroxymethylcellulose, Hydroxypropylmethylcellulose, Hydroxypropylcellulose, Hydroxyethylcellulose, Guaran, Xanthangummi, Polyvinylpyrrolidon, Poly(vinylpyrrolidon-Vinylacetat)-Copolymere oder eine Mischung derselben ist.

9. Magensaftresistent beschichtetes Pellet nach Anspruch 1, das einen Durchmesser im Bereich von 0,5 Millimetern bis 7 Millimetern aufweist.

10. Kapsel umfassend magensaftresistent beschichtete Pellets nach Anspruch 1.

11. Kapsel nach Anspruch 10, des Weiteren umfassend mindestens ein hydrophobes Material ausgewählt unter Talk, Magnesiumstearat, Stearinsäure, Glycerylbehenat, hydriertem Baumwollsamenöl, Trimyristin, Tripalmitin, Tristearin und pyrogenem Kieselgel.

12. Verfahren für die Herstellung von magensaftresistent beschichteten Pellets umfassend:
a) das Bereitstellen von Basispartikeln;
b) das Aufbringen einer aus aktiven Bestandteilen bestehenden Mischung und von Bindemittel auf die Basispartikel, wobei die aus aktiven Bestandteilen bestehende Mischung Folgendes umfasst:
i) eine Verbindung, die die Struktur 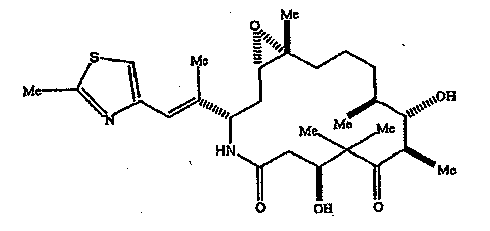 aufweist, oder ein pharmazeutisch akzeptables Salz, Solvat, Clathrat, Hydrat oder Prodrug derselben; und
ii) Lösungsmittel, Wasser oder eine Mischung davon;
c) Trocknen der Basispartikel unter Anwendung der aus aktiven Bestandteilen bestehenden Mischung, um beschichtete Partikel bereitzustellen; und
d) Aufbringen einer magensaftresistenten Beschichtung auf die beschichteten Partikel, um die magensaftresistent beschichteten Pellets bereitzustellen.

13. Verfahren nach Anspruch 12, wobei die aus aktiven Bestandteilen bestehende Mischung eine wässrige, aus aktiven Bestandteilen bestehende Suspension ist, die Partikel der Verbindung in Wasser umfasst.

14. Verfahren nach Anspruch 13, wobei die wässrige, aus aktiven Bestandteilen bestehende Suspension einen pH-Wert im Bereich von 6 bis 9 aufweist.

15. Verfahren nach Anspruch 14, wobei die wässrige, aus aktiven Bestandteilen bestehende Suspension des Weiteren Puffer umfasst.

16. Verfahren nach Anspruch 13, wobei die wässrige, aus aktiven Bestandteilen bestehende Suspension in Schritt b) auf die Basispartikel durch Sprühen in einem Wirbelbettsprühapparat aufgebracht wird.

17. Verfahren nach Anspruch 16, wobei die Basispartikel, auf die die wässrige, aus aktiven Bestandteilen bestehende Suspension aufgebracht wird, in Schritt c) bei einer Temperatur im Bereich von 25 °C bis 35 °C im Wirbelbettsprühapparat getrocknet werden.

18. Verfahren nach Anspruch 12, wobei das magensaftresistent beschichtete Pellet im Wesentlichen von Feuchtigkeit frei ist.

19. Verfahren nach Anspruch 12, wobei die Basispartikel in Schritt a) im Wesentlichen von Feuchtigkeit frei sind.

20. Magensaftresistent beschichtetes Pellet nach Anspruch 1 zur Verwendung bei der Behandlung von Krebs oder anderen Proliferationskrankheiten bei einem Säuger.

21. Magensaftresistent beschichtetes Pellet nach Anspruch 20, wobei der Säuger menschlich ist und die wirksame Menge der Verbindung oder eines pharmazeutisch akzeptablen Salzes, Solvats, Clathrats, Hydrats oder Prodrugs derselben im Bereich von 1 bis 500 mg/m² liegt.

22. Magensaftresistent beschichtetes Pellet nach Anspruch 20, wobei der Krebs Brustkrebs oder Lungenkrebs ist.

23. Kapsel umfassend mehrere der magensaftresistent beschichteten Pellets nach Anspruch 1 zur Verwendung bei der Behandlung von Krebs oder anderen Proliferationskrankheiten bei einem Säuger.

## Revendications

1. Bille à enrobage entérique comprenant:
a) une particule revêtue comprenant:
i) une particule de base; et
ii) une couche d'ingrédient actif disposée sur ladite particule de base, ladite couche d'ingrédient actif comprenant:
1) un composé ayant pour formule 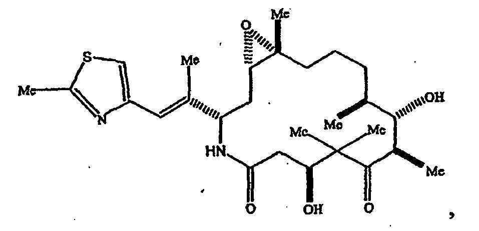 ou un de ses sels, solvates, clathrates, hydrates, ou promédicaments, pharmaceutiquement acceptables; et
2) au moins un liant; et
b) un enrobage entérique encapsulant ladite particule revêtue.

2. Bille à enrobage entérique selon la revendication 1 comprenant, sur la base du poids de ladite bille à enrobage entérique,
a) 10 à 80% en poids de ladite particule de base;
b) 0,1 à 50% en poids de ladite couche d'ingrédient actif; et
c) 5 à 55% en poids dudit enrobage entérique.

3. Bille à enrobage entérique selon la revendication 1, ladite bille à enrobage entérique étant pratiquement exempte d'humidité.

4. Bille à enrobage entérique selon la revendication 1 dans laquelle ledit au moins un liant est de l'amidon, de la gélatine, du saccharose, du glucose, du dextrose, de la mélasse, des dextrines modifiées, du lactose, de la gomme arabique, de l'alginate de sodium, de l'alginate de potassium, de la méthylcellulose, de l'hydroxyméthylcellulose, de l'hydroxypropylméthylcellulose, de l'hydroxypropylcellulose, de l'hydroxyéthylcellulose, de la gomme de guar, de la gomme de xanthane, de la polyvinylpyrrolidone, des copolymères de poly(vinylpyrrolidone-acétate de vinyle), ou un mélange de ceux-ci.

5. Bille à enrobage entérique selon la revendication 1 dans laquelle ledit enrobage entérique comprend du phtalate d'hydroxypropylméthylcellulose, du phtalate de polyacétate de vinyle, du phtalate d'acétate de cellulose, du succinate d'acétate d'hydroxypropylméthylcellulose, ou un copolymère d'acide méthacrylique.

6. Bille à enrobage entérique selon la revendication 1 comprenant en outre une sous-couche intercalée entre ladite couche d'ingrédient actif et ledit enrobage entérique.

7. Bille à enrobage entérique selon la revendication 6 comprenant de 0,1 à 10% en poids de ladite sous-couche, sur la base du poids de ladite bille à enrobage entérique.

8. Bille à enrobage entérique selon la revendication 6 dans laquelle ladite sous-couche est de l'amidon, de la gélatine, du saccharose, du glucose, du dextrose, de la mélasse, des dextrines modifiées, du lactose, de la gomme arabique, de l'alginate de sodium, de l'alginate de potassium, de la méthylcellulose, de l'hydroxyméthylcellulose, de l'hydroxypropylméthylcellulose, de l'hydroxypropylcellulose, de l'hydroxyéthylcellulose, de la gomme de guar, de la gomme de xanthane, de la polyvinylpyrrolidone, des copolymères de poly(vinylpyrrolidone-acétate de vinyle), ou un mélange de ceux-ci.

9. Bille à enrobage entérique selon la revendication 1 ayant un diamètre dans la gamme de 0,5 millimètre à 7 millimètres.

10. Capsule comprenant des billes à enrobage entérique selon la revendication 1.

11. Capsule selon la revendication 10 comprenant en outre au moins un matériau hydrophobe choisi parmi le talc, le stéarate de magnésium, l'acide stéarique, le béhénate de glycéryle, l'huile de coton hydrogénée, la trimyristine, la tripalmitine, la tristéarine, et la silice sublimée.

12. Procédé de préparation de billes à enrobage entérique, comprenant:
a) la fourniture de particules de base;
b) l'application d'un mélange d'ingrédients actifs et d'un liant sur lesdites particules de base, ledit mélange d'ingrédients actifs comprenant
i) un composé ayant comme structure: 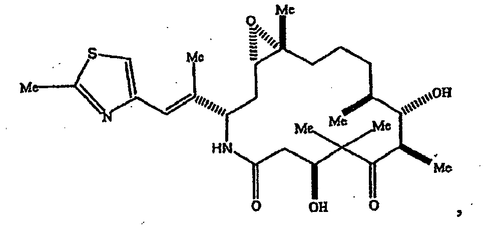 ou un de ses sels, solvates, clathrates, hydrates, ou promédicaments, pharmaceutiquement acceptables, et
ii) un solvant, de l'eau, ou un mélange des deux;
c) le séchage desdites particules de base ayant reçu une application dudit mélange d'ingrédients actifs pour fournir des particules revêtues; et
d) l'application d'un enrobage entérique sur lesdites particules revêtues pour fournir lesdites billes à enrobage entérique.

13. Procédé selon la revendication 12 dans lequel ledit mélange d'ingrédients actifs est une suspension aqueuse d'ingrédients actifs comprenant des particules dudit composé dans de l'eau.

14. Procédé selon la revendication 13 dans lequel ladite suspension aqueuse d'ingrédients actifs a un pH dans la gamme de 6 à 9.

15. Procédé selon la revendication 14 dans lequel ladite suspension aqueuse d'ingrédients actifs comprend en outre un tampon.

16. Procédé selon la revendication 13 dans lequel ladite suspension aqueuse d'ingrédients actifs est appliquée sur lesdites particules de base dans l'étape b) par pulvérisation dans un appareil à pulvériser en lit fluidisé.

17. Procédé selon la revendication 16 dans lequel lesdites particules de base ayant reçu une application de ladite suspension aqueuse d'ingrédients actifs sont séchées dans l'étape c) à une température dans la gamme de 25°C à 35°C dans ledit appareil à pulvériser en lit fluidisé.

18. Procédé selon la revendication 12 dans lequel ladite bille à enrobage entérique est pratiquement exempte d'humidité.

19. Procédé selon la revendication 12 dans lequel lesdites particules de base dans l'étape a) sont pratiquement exemptes d'humidité.

20. Bille à enrobage entérique selon la revendication 1 destinée à être utilisée dans le traitement d'un cancer ou d'autres maladies prolifératives chez un mammifère.

21. Bille à enrobage entérique selon la revendication 20 dans laquelle ledit mammifère est un humain et ladite quantité efficace se situe dans une gamme de 1 à 500 mg/m² dudit composé, ou d'un de ses sels, solvates, clathrates, hydrates, ou promédicaments, pharmaceutiquement acceptables.

22. Bille à enrobage entérique selon la revendication 20 dans laquelle ledit cancer est le cancer du sein ou le cancer du poumon.

23. Capsule comprenant une multitude desdites billes entériques selon la revendication 1, pour une utilisation dans le traitement d'un cancer ou d'autres maladies prolifératives chez un mammifère.
